# EUROPÄISCHE PATENTSCHRIFT

(11) **EP 0 338 098 B1**
(45) Veröffentlichungstag und Bekanntmachung des Hinweises auf die Patenterteilung: **04.08.1993**
(21) Anmeldenummer: 88106092.5
(22) Anmeldetag: 16.04.1988
(51) Int. Cl.: C12M 1/12, C12N 1/02

(54) **Verfahren zur Erzeugung von Zellmaterial aus Mikroorganismen, insbesondere von Hefen**
Process for obtaining cellular material from microorganisms, especially from yeasts
Procédé d'obtention de matériel allulaire à partir de micro-organismes, en particulier de levures

(43) Veröffentlichungstag der Anmeldung: 25.10.1989
(73) Patentinhaber: Starcosa GmbH, D-38122 Braunschweig (DE)
(72) Erfinder: Tegtmeier, Uwe, Dr., D-3341 Wittmar (DE)
(74) Vertreter: Einsel, Martin Dipl.-Phys.

(56) Entgegenhaltungen:
- EP-A- 0 017 853
- EP-A- 0 229 872
- DE-A- 973 185

## Beschreibung

Die Erfindung betrifft ein Verfahren zur Erzeugung von Zellmaterial aus Mikroorganismen, insbesondere von Hefen, gemäß dem Gattungsbegriff des Anspruches 1.

Es sind Verfahren zur fermentativen Erzeugung von Nähr- und Futterhefe als Eiweißträger zur Beimischung zu menschlichen Nahrungsmitteln oder tierischen Futtermitteln bekannt, bei denen eine mehrstufige Fermentation erfolgt. Dabei werden Hefen der Gattungen Pichia, Hansenula oder Torulopsis verwendet. Diese Hefen können die meisten Hexosen und Pentosen sowie einige Disaccharide verwerten. Als technische Substrate eignen sich insbesondere Melasse, verzuckerte Stärkehydrolysate, hydrolisiertes Zellulose bzw. Hemizellulose enthaltendes Material, wie Holz oder Stroh, Abwässer der Papierindustrie und andere Zucker enthaltende Reststoffe. Zur Bildung der Zellmasse ist ein hoher spezifischer Sauerstoffeintrag bei der Fermentation erforderlich.

Mit dem bekannten Verfahren sind lediglich Produktströme mit einer niedrigen Konzentration des Zellmaterials erreichbar. Diese Konzentration liegt in Abhängigkeit von den Substraten bei ca. 10 bis 60 g Biomasse-Trockensubstanz (BTS) je Liter.

Zur Reduzierung des Aschegehaltes und zur Verbesserung der Farbe des durch das mehrstufige Fermentationsverfahren gewonnenen Produktes ist es erforderlich, die in dem aus dem Fermenter ablaufenden Stoffstrom enthaltene Biomasse zunächst zu konzentrieren und danach mehrfach mit Frischwasser zu waschen und zwischen den Waschprozessen wiederum zu konzentrieren. Das Konzentrieren und Waschen wird dabei mittels Separatoren ausgeführt, wobei dem zulaufenden Strom jeweils eine bestimmte Menge Frischwasser zugegeben wird und als Dickphase das jeweils konzentrierte Zellmaterial erhalten wird.

Bei dem bekannten Verfahren sind aufgrund der kurzen Verweilzeiten in den Separatoren erhebliche Frischwassermengen erforderlich. Die jeweiligen Klarphasenabläufe der Separatoren stellen Abwasserströme dar, welche eine Rückführung zur Fermentation wegen der darin enthaltenen Infektionen allenfall nach aufwendiger thermischer Behandlung ermöglichen. Wenn durch Rückführung des Abwassers zur Fermentation die Frischwassermengen reduziert werden sollen, ergibt sich durch die aufwendige thermische Behandlung ein entsprechend hoher Energiebedarf für die Aufbereitung des Abwassers.

Bei den obengenannten geringen Zellmassekonzentrationen ergeben sich auch Schwierigkeiten, den Prozeß biologisch stabil zu betreiben. Zu diesem Zweck ist es erforderlich, das verwendete Substrat zunächst einer energetisch aufwendigen Behandlung durch Klären und Pasteurisieuren zu unterwerfen und dabei bereits die Kolloide zu entfernen, die anderenfalls die Waschungen noch aufwendiger gestalten würden.

Die aus der letzten Waschstufe gewonnene Dickphase (Zellmaterial) fällt bestenfalls mit ca. 15 bis 16% BTS an und wird anschließend nach einer Autolysebehandlung in einer Eindampfanlage auf Werte von ca. 25% BTS eingedickt. Dieses Konzentrat wird schließlich in einem Sprühtrockner schonend getrocknet, ehe seine Weiterverwertung erfolgen kann.

Der Erfindung liegt die Aufgabe zugrunde, ein Verfahren nach dem Gattungsbegriff des Anspruches 1 so weiterzubilden, daß bei geringem Energiebedarf eine hohe Biomassekonzentration und biologische Stabilität des Verfahrensablaufes erreicht wird.

Zur Lösung vorstehender Aufgabe kennzeichnet sich das einleitend genannte Verfahren erfindungsgemäß durch die im Kennzeichen des Anspruches 1 genannten Merkmale.

Durch die vorgenannten Verfahrensschritte wird eine Rückhaltung und Aufkonzentrierung des Zellmaterials in der einstufigen, kontinuierlichen Fermentation sowie der nachgeschalteten Waschstufe dadurch erreicht, daß eine definierte Trennung der Flüssigphase in der Mikrofiltrationsstufe erfolgt. Da des Permeat aus der der Waschstufe zugeordneten Mikrofiltrationsstufe mikroorganismenfrei ist, kann es ohne Erhöhung der Infektionsgefahr in den Fermenter zurückgeführt werden. Durch diese Verfahrensweise wird das zur Waschung benötigte Frischwasser über die Permeatrückführung als Prozeßwasser im Fermenter verwendet.

In der Fermentation wird durch die Zellrückhaltung mittels der dieser nachgeordneten Mikrofiltrationsstufe eine konzentrierte Arbeitsweise des Prozesses erreicht, die einhergeht mit einer Stabilisierung der mikrobiologischen Verhältnisse, so daß eine vorherige Aufarbeitung der Substrate nicht erforderlich ist.

Mit dem neuen Verfahren lassen sich Biomassekonzentrationen von 90 bis 120 g BTS/l einstellen. Durch die hohe Biomassekonzentration und die Aufkonzentrierung an osmotisch wirksamen Komponenten im Fermentationsfluid wird ein negativer Selektionsmechanismus hinsichtlich der Fremdkeime bewirkt. Hierdurch werden bakterielle Infektionen unterdrückt.

Die Anreicherung von osmotisch wirksamen Substanzen führt zu einer Steigerung der intrazellulären Trockensubstanz, durch die sich die nachfolgenden Prozeßschritte vereinfachen lassen.

Die Mikrofiltration im Anschluß an die Fermentation sowie an die Waschstufe wird durch die hohe Biomassenkonzentration nicht nachteilig beeinträchtigt. Haupteinflußgröße für die Trennung durch die Mikrofiltration ist die Viskosität der Flüssigphase. Diese Viskosität, welche durch die gelösten Komponenten bestimmt wird, kann bei dem beschriebenen Verfahren sehr niedrig gehalten werden.

In der Waschstufe kann eine vorbestimmte Verweilzeit der Biomasse eingestellt werden. Das Verhältnis von Biomassestrom zum Waschwasserstrom kann durch die Wahl des jeweiligen Permeatstromes eingestellt und dadurch der Wascheffekt genau definiert werden. Durch die möglichen langen Verweilzeiten der Biomasse in der Waschstufe sind nur geringe Waschwassermengen erforderlich.

Innerhalb der Waschstufe kann die Zellkonzentration von ca. 20% BTS/l eingehalten werden, wodurch der Energiebedarf bei der anschließenden Eindampfung entsprechend reduziert wird. Dabei eignet sich das aus diesem Verfahren erhaltene Zellmaterial wegen der höheren intrazellularen Trockensubstanzgehalte für eine Aufkonzentrierung in der Eindampfanlage auf höhere Endtrockensubstanzgehalte.

Das neue Verfahren kann weitgehend automatisiert werden. Manuelle Arbeitsschritte, wie z.B. das Reinigen von Separatoren, entfallen.

Statt mit nur einer Waschstufe zu arbeiten, kann auch gemäß Anspruch 2 verfahren werden, indem der aus dem Fermenter abgeführte Stoffstrom nacheinander durch mehrere Waschstufen mit diesen zugeordneten Mikrofiltrationsstufen geleitet wird. Dabei wird gemäß Anspruch 2 die Waschflüssigkeit ausschließlich in die letzte Waschstufe eingeleitet und aus der der Fermentation nachgeordneten ersten Waschstufe als Permeatstrom dem Fermenter als Prozeßwasser zugeleitet.

Zweckmäßig ist es, wenn als Waschflüssigkeit zusatzfreies und unbehandeltes Wasser (Trinkwasser) verwendet wird und wenn ausschließlich der Permeatstrom aus der dem Fermenter nachgeordneten Mikrofiltrationsstufe als Abwasser abgeführt wird.

Um für das Zellwachstum den notwendigen Sauerstoff zur Verfügung zu stellen, ist es erforderlich, einen möglichst hohen Eintrag an Sauerstoff in die Fermentation zu gewährleisten. Die in das Fermentationsfluid einzubringende Sauerstoffmenge sollte bei dem neuen Verfahren mindestens 35 kg/m³ Std., bezogen auf die Fluidmasse, betragen. Die derzeit höchste eintragbare Sauerstoffmenge liegt bei 45 bis 50 kg/m³ Std., bezogen auf die Fluidmasse.

Um die Infektionsgefahr möglichst gering zu halten, empfiehlt es sich, den pH-Wert des Fermentationsfluids in dem Fermenter auf höchstens 4,5, vorzugsweise zwischen 3,5 und 4, einzustellen und aufrechtzuerhalten.

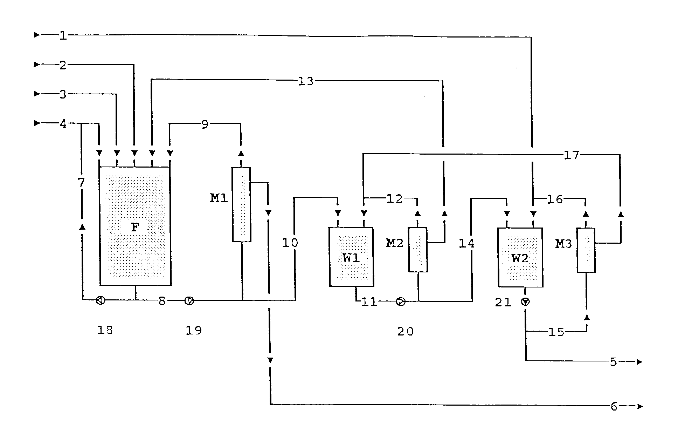

Die Zeichnung gibt ein Verfahrensschema der Erfindung an einem Ausführungsbeipsiel in schematischer Darstellung wieder. Dabei sind lediglich die Fermentation, sowie die Waschstufen dargestellt, während die nachfolgenden Prozeßschritte, wie Eindampfen und Trocknen, nicht näher wiedergegeben und erläutert sind.

Dem in der Zeichnung wiedergegebenen Fermenter F, in welchem das Wachstum der Mikroorganismen stattfindet, werden die hierzu benötigten und bekannten Wuchs- sowie Nährstoffe über die Leitung 3 zugeführt. Ferner wird in den Fermenter F über die Leitung 2 das Zucker enthaltende Substrat, beispielsweise Melasse, eingeleitet. Der für das Wachstum benötigte Sauerstoff wird mittels einer besonderen, mit einer Mehrphasendüse ausgerüsteten Belüftungsvorrichtung aus der Zuleitung 4 angesaugt. Dabei wird der Treibstrahl mittels einer Pumpe 18 erzeugt, die saugseitig mit dem Fermenter F und druckseitig über die Leitung 7 mit der Leitung 4 verbunden ist.

Durch eine Leitung 8 und eine darin angeordnete Pumpe 19 wird der Stoffstrom aus dem Fermenter F dosiert abgezogen und teils der Mikrofiltrationseinheit M1 und teils über die Leitung 10 in die erste Waschstufe W1 überführt. Der Retentatstrom aus der Mikrofiltrationsstufe M1 wird über die Leitung 9 wieder in den Fermenter F zurückgeleitet.

In dem dargestellten Beispiel sind zwei Waschstufen W1 und W2 vorgesehen. Der Waschstufe W1 wird das Permeat aus der Mikrofiltrationseinheit 3 der Waschstufe W2 als Waschflüssigkeit zugeleitet. Der Ablauf der Waschstufe W1 wird über die Leitung 11 und die Pumpe 20 teils in die Mikrofiltrationseinheit M2 und teils über die Leitung 14 der Waschstufe W2 zugeführt. Das Retentat aus der Mikrofiltrationsstufe M2 wird über die Leitung 12 wieder in die Waschstufe W1 zurückgeführt, während das Permeat aus dieser Mikrofiltrationsstufe M2 über die Leitung 13 in den Fermenter F geleitet wird.

Über die Leitung 1 wird der Waschstufe W2 das Waschwasser zugeleitet. Der Ablauf aus der Waschstufe W2 wird über die Pumpe 21 teils über die Leitung 15 in die Mikrofiltrationsstufe M3 gepumpt und teils über die Leitung 5 als biomassehaltiger Endablauf den nachfolgenden Prozeßschritten, wie Eindampfung und Trocknung, zugeleitet.

Aus der Mikrofiltrationsstufe M3 wird das Retentat über die Leitung 16 der Waschstufe W2 zugeleitet, während das Permeat dieser Mikrofiltrationsstufe M3 in die Waschstufe W1 geleitet wird.

Aus der Mikrofiltrationsstufe M1 wird das Permeat über die Leitung 6 als Abwasserstrom abgeleitet.

Das Verfahrensschema zeigt deutlich die Entkoppelung von Flüssig- und Festphase mittels der Mikrofiltrationseinheit M1 bis M3, wodurch die obengenannten vorteilhaften Wirkungen entstehen, durch die gegenüber dem Stand der Technik eine wesentlich bessere Verfahrensführung mit höherem Feststoffgehalten des biomassehaltigen Endablaufes und höheren Trockensubstanzgehalt der Zellen erreicht werden kann.

Nachstehend wird ein Verfahrensbeispiel für eine kontinuierliche, einstufige fermentative Erzeugung von Futterhefe mit einer Anlage der vorstehend beschriebenen Art angegeben.

In einem Fermenter mit 100 l Gesamtvolumen und 29 l Arbeitsvolumen wurde bei einer aktuellen Hefetrockensubstanz (HTS) von 100 g/l eine Biomassebildung durchgeführt. Eingesetzt wurde der Hefestamm ATCC 9256. Für die Fermentation wurden 3,9 kg/h Substrat in Form verdünnter Melasselösung mit 25% Saccharose sowie 1,5 kg/h einer 10%igen Nährsalzlösung aus Diammoniumphosphat, Magnesiumsulphat und Harnstoff dem Fermenter zugeleitet. Außerdem waren geringe Mengen an Schwefelsäure zur Einstellung des pH-Wertes auf ca. 4,0 bis 4,2 notwendig. Der Sauerstoffbedarf wurde durch Zuführung von ca. 6,2 Nm³/h Luft gedeckt. Sowohl die Luft als auch das Substrat waren nicht vorbehandelt.

Die Fermentation wurde bei einer Temperatur von 34 bis 35°C durchgeführt. Für den Lufteintrag wurde ein Treibstrahl von 1 m³/h erzeugt.

Für den Betrieb der Mikrofiltrationseinheit M1, die in diesem Fall aus 1 Modul MD 080 CP 2N der Fa. Enka AG, Wuppertal bestand, wurde ein Retentatstrom von 1 m³/h erzeugt, um einen Permeatstrom von ca. 20,9 l/h zu erreichen. Zu der Waschstufe W1 gelangte ein biomassehaltiger Strom von 5,2 l/h.

Als Prozeßwasser wurden 15,6 l/h unbehandeltes Wasser in die Waschstufe W2 eingeführt. Das aus der Mikrofiltrationseinheit M3 abgeführte Permeat mit 16,7 l/h wurde ebenfalls zur Waschstufe W1 geführt. Die Mikrofiltrationseinheit M2 und M3 waren ebenfalls mit dem oben bezeichneten Modultyp ausgestattet.

Aus der Mikrofiltrationseinheit M2 wurden 20 l/h Permeat abgezogen und dem Fermenter F als Verdünnungswasser zugesetzt. Die Retentatströme der Mikrofiltrationseinheiten M2 und M3 betrugen ca. 1 m³/h.

Von der Waschstufe W1 wurden ca. 4,4 l/h zellmassehaltiger Ablaufstrom zur Waschstufe W2 geleitet.

Als Abwasserstrom in Form des Permeats von der Mikrofiltrationseinheit M1 mußten 18,4 l/h entfernt werden. Der zellmaterialhaltige Produktstrom, welcher aus der Waschstufe W2 abgezogen wurde, betrug ca. 2,6 l/h mit einer Hefetrockensubstanz von 20%.

Somit wurde für die Erzeugung von 12,5 kg HTS/Tag eine Frischwassermenge von 374,4 l/Tag benötigt. Bei einer Reduzierung der maximalen Waschstumsrate mit einhergehender Vergrößerung des benötigten Fermentervolumens läßt sich dieser Wert noch erheblich reduzieren. Bei einem spezifischen Fermentervolumen von ca. 3 l/kg HTS Tag ist eine solche Volumenvergrößerung realistisch.

Eine Betrachtung des spezifischen Eintrages an elektrischer Energie ist bei einer Anlagengröße, wie hier verwendet, nicht möglich. Berechnungen zeigen, daß bei einer Tagesproduktion von ca. 10 t HTS ein spezifischer Energieeintrag von 380 kWh/t HTS resultiert.

Thermische Energie wurde nicht benötigt.

Das aus der letzten Waschstufe W2 abgezogene Produkt wurde anschließend autolysiert und sprühgetrocknet, bei Maximaltemperaturen von 80°C. Es zeigte sich, daß die Farbwerte vergleichbar waren mit einer sehr guten Trockenbackhefe. Der Aschegehalt war niedriger als 6,2, der Eiweißgehalt betrug 49,4% auf TS.

Bei der Erzeugung von Hefen für die menschliche Ernährung muß anders als bei Hefen für Futterzwecke das zu verarbeitende Substrat aufbereitet werden, um ein möglichst bakterienarmes Endprodukt zu erhalten.

Das einleitend beschriebene Verfahren nach den Ansprüchen 1 bis 4 ist auch geeignet zur Erzeugung von Bakterien für Nahrungs- und/oder Futtermittelzwecke, wobei in diesem Falle jedoch das Substrat zuvor sterilisiert werden muß und auch eine sterile Prozeßführung erforderlich ist. Außerdem muß bei der Bakterienherstellung mit einem pH-Wert im Fermenter von etwa 5,5 bis 6,5 gearbeitet werden. Auch bei der Bakterienherstellung werden die gleichen Vorteile erreicht, die im Zusammenhang mit der Erzeugung von Hefen im einzelnen angegeben sind.

## Patentansprüche

1. Verfahren zur Erzeugung von Zellmaterial aus Mikroorganismen, insbesondere von Hefen als Nahrungs- und/oder Futtermittel oder zur Herstellung von bzw. zur Beimengung zu Nahrungs- und/oder Futtermitteln, durch aerobes Wachstum der Mikroorganismen auf zuckerhaltigen Substraten, wie Melasse, in einer einstufigen kontinuierlichen Fermentation mit nachfolgendem Separieren und Aufkonzentrieren der aus dem Stoffstrom des Fermenters gewonnenen Biomasse sowie anschließendes Waschen und erneutes Aufkonzentrieren dieser Biomasse, **dadurch gekennzeichnet,**
a) daß der aus dem Fermenter ablaufende Stoffstrom teils einer Mikrofiltrationsstufe, teils einer Waschstufe zugeleitet und das Retentat aus der Mikrofiltrationsstufe in den Fermenter zurückgeführt wird,
b) daß der Ablauf der Waschstufe teils einer weiteren Mikrofiltrationsstufe und teils einer weiteren Behandlung zugeführt wird,
c) daß der Permeatstrom der der Waschstufe zugeordneten Mikrofiltrationsstufe in den Fermenter zurückgeführt wird und
d) daß die Waschflüssigkeit in einer für den Prozeßablauf benötigten Menge in die Waschstufe eingeleitet wird.

2. Verfahren nach Anspruch 1, **dadurch gekennzeichnet,** daß der aus dem Fermenter in die Waschstufe überführte Stoffstrom nacheinander durch mehrere Waschstufen mit diesen zugeordneten Mikrofiltrationsstufen geleitet wird, daß der Ablauf einer jeden Waschstufe teils der nachfolgenden Mikrofiltration und teils der nächstfolgenden Waschstufe zugeleitet wird, daß der Permeatstrom aus der Mikrofiltration des Ablaufes der zweiten und folgenden Waschstufe jeweils der vorhergehenden Waschstufe zugeleitet wird und daß die Waschflüssigkeit in einer für den Prozeßablauf benötigten Menge in die letzte Waschstufe eingeleitet wird.

3. Verfahren nach Anspruch 1 oder 2, **dadurch gekennzeichnet,** daß als Waschflüssigkeit zusatzfreies und unbehandeltes Wasser verwendet und ausschließlich der Permeatstrom aus der dem Fermenter nachgeordneten Mikrofiltrationsstufe als Abwasser abgeführt wird.

4. Verfahren nach einem der Ansprüche 1 bis 3, **dadurch gekennzeichnet,** daß dem Fermentationsfluid in dem Fermenter eine Sauerstoffmenge von mindestens 35 kg/m³ Std., bezogen auf die Fluidmasse, zugeführt wird.

5. Verfahren nach einem der Ansprüche 1 bis 4, **dadurch gekennzeichnet,** daß der pH-Wert des Fermentationsfluids in dem Fermenter auf höchstens 4,5, vorzugsweise zwischen 3,5 und 4, eingestellt und aufrechterhalten wird.

## Claims

1. A process for the production of cellular material from micro-organisms - in particular of yeasts as a foodstuff and/or animal feed or for manufacture of and/or admixing with foodstuffs and/or animal feeds - by aerobic growth of the microorganisms on sugar-containing substrates - such as molasses - in a single-stage continuous fermentation with subsequent separation and further concentration of the biomass obtained from the substance stream of the fermenter as well as subsequent washing and further concentration once again of this biomass, characterised in that
a) the substance stream issuing from the fermenter is fed partly to a microfiltration stage partly to a washing stage and the substance retained from the microfiltration stage is returned to the fermenter,
b) the discharge from the washing stage is supplied partly to a further microfiltration stage and partly to a further treatment,
c) the permeate flow from the microfiltration stage associated with the washing stage is returned to the fermenter and
d) the washing liquid is introduced into the washing stage in a quantity necessary for the procedure.

2. A process in accordance with Claim 1, characterised in that the substance stream conveyed from the fermenter into the washing stage is consecutively conducted through a plurality of washing stages with microfiltration stages associated therewith, in that the discharge of each washing stage is fed partly to the subsequent microfiltration and partly to the next washing stage, in that the permeate flow from the microriltration of the discharge of the second and following washing stage is in each case fed to the preceding washing stage, and in that the washing liquid is introduced into the final washing stage in a quantity necessary for the procedure.

3. A process in accordance with Claim 1 or 2, characterised in that additive-free and untreated water is used as a washing liquid and solely the permeate flow from the microfiltration stage arranged after the fermenter is drawn off as waste liquid.

4. A process in accordance with any one of Claims 1 to 3, characterised in that an oxygen quantity of at least 35 kg/m³ per hour, relative to the fluid mass, is supplied to the fermentation fluid in the fermenter.

5. A process in accordance with any one of Claims 1 to 4, characterised in that the pH-value of the fermentation fluid in the fermenter is adjusted to 4.5 at most, preferably between 3.5 and 4, and is maintained thereat.

## Revendications

1. Procédé pour produire un matériau cellulaire à partir de microorganismes, en particulier de levures pour produits alimentaires et/ou aliments pour animaux, ou pour la réalisation et/ou le mélange avec des produits alimentaires et/ou d'aliments pour animaux, par une croissance aérobie de microorganismes sur des substrats contenant du sucre tels que de la mélasse, par une fermentation continue à étage unique avec séparation subséquente et concentration de la biomasse obtenue du courant de produit provenant du fermentateur ainsi qu'un nouveau lavage et une nouvelle concentration de cette biomasse, caractérisé en ce que:
a) le courant de produit qui provient du fermentateur est dirigé en partie vers un étage de microfiltration et en partie vers un étage de lavage et le rétentat est renvoyé de l'étage de microfiltration vers le fermentateur,
b) le courant de sortie de l'étage de lavage est renvoyé en partie à un autre étage de microfiltration et en partie à un autre traitement;
c) le courant de perméat de l'étage de microfiltration qui est associé à l'étage de lavage est renvoyé dans le fermentateur et
d) le liquide de lavage est envoyé dans l'étage de lavage selon une quantité nécessaire au déroulement du procédé.

2. Procédé selon la revendication 1, caractérisé en ce que le courant de produit qui est envoyé du fermentateur à l'étage de lavage est dirigé successivement par plusieurs étages de lavage avec un étage de microfiltration associé à chacun d'eux, en ce que le courant de sortie de chacun des étages de lavage est dirigé en partie vers la microfiltration qui suit et en partie vers l'étage de lavage qui suit, en ce que le courant de perméat provenant de la microfiltration du courant de sortie du second étage de lavage et des suivants est dirigé vers l'étage de lavage précédent respectif, et en ce que le liquide de lavage est introduit dans le dernier étage de lavage selon la quantité nécessaire au déroulement du procédé.

3. Procédé selon la revendication 1 ou 2, caractérisé en ce qu'on utilise en tant que liquide de lavage de l'eau sans adjuvants et non traitée, et en ce que le courant de perméat est dirigé exclusivement depuis le fermentateur vers l'étage de microfiltration qui suit en tant qu'eau usée.

4. Procédé selon l'une quelconque des revendications 1 à 3, caractérisé en ce qu'une quantité d'oxygène d'au moins 35 kg/m³ par heure, rapportée à la masse du liquide, est dirigée dans le fermentateur.

5. Procédé selon l'une quelconque des revendications 1 à 4, caractérisé en ce que la valeur du pH du liquide de fermentation dans le fermentateur est réglée sur 4,5 et de préférence entre 3,5 et 4, et est maintenue à ce niveau.
